# EUROPEAN PATENT APPLICATION

(11) **EP 3 231 441 A1**
(43) Date of publication of application: **18.10.2017**
(21) Application number: 15868210.4
(22) Date of filing: 10.12.2015
(51) Int. Cl.: A61K 39/00, A61K 39/385, A61K 39/39, C07K 7/08, C07K 14/005

(54) **NOVEL PROTEIN STRUCTURE PRODUCED BY EFFECTIVE ANTIBODY USED FOR IMMUNIZATION**

(30) Priority: 10.12.2014 US 201462090180 P
(71) Applicant: Vaxsia Biomedical Inc., Taipei City 11503 (TW)
(72) Inventor: KAN, Ming Chung, New Taipei City 24249 (TW)
(74) Representative: Michalski Hüttermann & Partner Patentanwälte mbB
(86) International application number: PCT/CN2015/096949
(87) International publication number: WO 2016/091181

(57) **Abstract**

A method for enhancing an immune-response, the method comprising covalently bonding an amphiphilic peptide to an antigen to form an antigen complex compound, and administering the antigen complex compound to a subject. The antigen compound having an amphiphilic helical structure can enhance antigen delivery.

## Description

### BACKGROUND OF THE INVENTION

### CROSS-REFERENCE TO RELATED APPLICATIONS

The present application claims the priority of U.S. Provisional Patent Application No. 62/090,180 filed on December 10, 2014, the disclosure of all of which are incorporated by reference herein in their entirety.

### Field of the Invention

The exemplary embodiment(s) of the present invention relates to a field of the method of improving immune response. More specifically, the exemplary embodiment(s) of the present invention relates to a method for improving immune response by using an antigen-complex with amphipathic helix structure.

### Description of Related Art

Many microbe proteins that are required for infectivity have been identified and mentioned to be potential vaccine candidates with the far-going researches on microbe infection mechanisms. These vaccine candidates embrace various molecular types including peptides, proteins, glycoproteins, and polysaccharides from a pathogenic microbe. However, the lack of antigenicity of these candidates when produced by synthesis or purification prevents these vaccine candidates from serving as useful vaccine.

Hence, there are several well-known methods to increase the antigenicity of these candidates, including addition of adjuvant in vaccine formulation or by fusing these candidates to an immunogen to increase the antigenicity of these candidates. However, adjuvant usually induces innate immune responses that cause the swelling and pain on the injection site. Further, the variability of responses in population to a specific adjuvant component causes mixed results of the vaccination and serious adverse events from high level of adjuvant addition.

Several immunogens have been developed to be used to fuse with potential vaccine candidates to increase their antigenicity. For examples, Hepatitis B core antigen HBcAg, Pertussis toxin CyaA and exotoxin from *E*.*coli* have been used to increase the antigenicity of attached protein through different mechanisms. However, due to the large size of these immunogens, the insertion or fusion of a heterologous protein often interferes with the proper folding of the fusion protein. Although misfolded proteins can be refolded with several procedures, it actually increases the production cost. Hence, a small peptide that enables high antigenicity of fused protein and has no interference on protein folding is highly desired.

On the other hand, in the specific immune response, T effector cell responses are important for the establishment of memory T cells and memory B cells. One of the T effector cell responses are type 2 T helper cell responses where naïve CD4+ T cell is stimulated by the T cell epitope bound on MHC class II molecules on the surface of antigen presenting cell. These epitopes are derived from the antigen processed in the endosomal pathway where they are bound with MHC class II molecules and exported to the cell surface. Thus delivery of antigen through the endosomal pathway in antigen presenting cells is a criteria for stimulating type 2 T helper responses, or Th2 responses. Th2 responses are characterized by enhancing IgG class switch toward IgG1 subtype. On the other hand, Th1 responses enhance the IgG class switch toward IgG2a and IgG3. Thus the ratio between IgG1 titer and IgG2q/IgG3 titer can be used as a surrogate marker for the type of T effector responses induced by a specific immunogen.

### SUMMARY OF THE INVENTION

The present invention provides a method of enhancing immune response, comprising combining an amphipathic peptide to an antigen to form an antigen-complex composition; and administering the antigen-complex to a subject.

In one embodiment, the present invention provides the method of enhancing immune response, wherein the amphipathic peptide may range from 4 to 45 amino acids.

In one embodiment, the present invention provides the method of enhancing immune response, wherein the amphipathic peptide may comprise an amino acid sequence of SEQ ID NO: 1, an amino acid sequence of SEQ ID NO: 2, or an amino acid sequence of SEQ ID NO: 3.

In another embodiment, the present invention provides the method of enhancing immune response, wherein the amphipathic peptide comprises an amino acid sequence including a A₁B₁B₂A₂ and/or A₃B₃B₄B₅A₄ motif, wherein A₁, A₂, A₃ and A₄ are each individually selected from the group consisting of arginine (R) and lysine (K); B₁ is selected from the group consisting of lysine (K), arginine (R), isoleucine (I), leucine (L), and valine (V); B₂ is selected from the group consisting of isoleucine (I), leucine (L), valine (V), phenylalanine (F), tyrosine (Y), and tryptophan (W); B₃ is selected from the group consisting of, isoleucine (I), leucine (L), and valine (V); B₄ is selected form the group consisting of phenylalanine (F), tryptophan (W) and tyrosine (Y); and B₅ is selected from the group consisting of phenylalanine (F), tyrosine (Y), tryptophan (W), threonine (T), cysteine (C) and serine (S).

In another embodiment, the present invention provides the method of enhancing immune response, wherein the amphipathic peptide may comprise an amino acid sequence of SEQ ID NO: 4, an amino acid sequence of SEQ ID NO: 5, an amino acid sequence of SEQ ID NO: 6, an amino acid sequence of SEQ ID NO: 7, amino acid sequence of SEQ ID NO: 8, an amino acid sequence of SEQ ID NO: 9, an amino acid sequence of SEQ ID NO: 10, or an amino acid sequence of SEQ ID NO: 11.

In another embodiment, the present invention provides the method of enhancing immune response, wherein the antigen may be a part of a pathogenic agent selected from the group consisting of Influenza virus of Type A, Influenza virus of Type B, Influenza virus of Type C, Dengue virus, Human papillomavirus, Hepatitis A virus, Hepatitis B virus, Hepatitis C virus, Varicella Zoster virus, Cytomegalovirus, Ebola virus, *Haemophilus influenzae* type b, *Helicobacter pylori,* Herpes simplex virus 1 and 2, *Plasmodium* species, Measles virus, Middle East respiratory syndrome coronavirus, Mumps virus, *Bordetella pertussis,* Poliovirus, Rabies virus, Respiratory syncytial virus, Rotavirus, Rubella virus, SARS coronavirus, *Clostridium tetani, Mycobacterium tuberculoses,* West Nile virus, *Streptococcus pneumoniae, , Staphylococcus aureus, Clostridium difficile, Neisseria meningitidis* and *Salmonella enterica* subsp. *enterica.*

In another embodiment, the present invention provides the method of enhancing immune response, wherein the antigen may be selected from the group consisting of oligosaccharide, polypeptide, protein, polysaccharide, peptide and a combination thereof.

In another embodiment, the present invention provides the method of enhancing immune response, wherein the subject may be warm-blooded animals.

The present invention provides an antigen-complex composition, comprising: a A₁B₁B₂A₂ and/or A₃B₃B₄B₅A₄ motif; a first peptide, linking to the N-terminal of the A₁B₁B₂A₂ and/or A₃B₃B₄B₅A₄ motif; a second peptide, linking to the C-terminal of the A₁B₁B₂A₂ and/or A₃B₃B₄B₅A₄ motif; and an antigen, covalently linked to the first peptide or the second peptide, wherein: A₁, A₂, A₃ and A₄ are each individually selected from the group consisting of arginine (R) and lysine (K); B₁ is selected from the group consisting of lysine (K), arginine (R), isoleucine (I), leucine (L), and valine (V); B₂ is selected from the group consisting of isoleucine (I), leucine (L), valine (V), phenylalanine (F), tyrosine (Y), and tryptophan (W); B₃ is selected from the group consisting of, isoleucine (I), leucine (L), and valine (V); B₄ is selected form the group consisting of phenylalanine (F), tryptophan (W) and tyrosine (Y); and B₅ is selected from the group consisting of phenylalanine (F), tyrosine (Y), tryptophan (W), threonine (T), cysteine (C) and serine (S); and the A₁B₁B₂A₂ and/or A₃B₃B₄B₅A₄ motif, the first peptide and the second peptide form an amphipathic helix secondary structure.

In another embodiment, the present invention provides the antigen-complex composition wherein the first peptide may range from 0 to 20 amino acids and the second peptide may range from 0 to 20 amino acids.

In another embodiment, the present invention provides the antigen-complex composition wherein the antigen may be selected from the group consisting of oligosaccharide, polypeptide, protein, polysaccharide, peptide and a combination thereof.

The present invention provides a method of improving antigen delivery, comprising administering an antigen-complex composition to a subject, wherein the antigen-complex composition comprises: a A₁B₁B₂A₂ and/or A₃B₃B₄B₅A₄ motif; a first peptide, linking to the N-terminal of the A₁B₁B₂A₂ and/or A₃B₃B₄B₅A₄ motif; a second peptide, linking to the C-terminal of the A₁B₁B₂A₂ and/or A₃B₃B₄B₅A₄ motif; and an antigen, covalently linked to the first peptide or the second peptide, wherein: A₁, A₂, A₃ and A₄ are each individually selected from the group consisting of arginine (R) and lysine (K); B₁ is selected from the group consisting of lysine (K), arginine (R), isoleucine (I), leucine (L), and valine (V); B₂ is selected from the group consisting of isoleucine (I), leucine (L), valine (V), phenylalanine (F), tyrosine (Y), and tryptophan (W); B₃ is selected from the group consisting of, isoleucine (I), leucine (L), and valine (V); B₄ is selected form the group consisting of phenylalanine (F), tryptophan (W) and tyrosine (Y); and B₅ is selected from the group consisting of phenylalanine (F), tyrosine (Y), tryptophan (W), threonine (T), cysteine (C) and serine (S); and the A₁B₁B₂A₂ and/or A₃B₃B₄B₅A₄ motif, the first peptide and the second peptide form an amphipathic helix secondary structure.

In an embodiment, the present invention provides the method of improving antigen delivery, wherein the first peptide may range from 0 to 20 amino acids and the second peptide may range from 0 to 20 amino acids.

In one embodiment, the present invention provides the method of improving antigen delivery, wherein the antigen may be selected from the group consisting of oligosaccharide, polypeptide, protein, polysaccharide, peptide and a combination thereof.

The present invention provides an amphipathic peptide comprising an amino acid sequence of SEQ ID NO: 1, an amino acid sequence of SEQ ID NO: 2, an amino acid sequence of SEQ ID NO: 3, an amino acid sequence of SEQ ID NO: 4, an amino acid sequence of SEQ ID NO: 5, an amino acid sequence of SEQ ID NO: 6, an amino acid sequence of SEQ ID NO: 7, an amino acid sequence of SEQ ID NO: 8, an amino acid sequence of SEQ ID NO: 9, an amino acid sequence of SEQ ID NO: 10, or an amino acid sequence of SEQ ID NO: 11.

With these and other objects, advantages, and features of the invention that may become hereinafter apparent, the nature of the invention may be more clearly understood by reference to the detailed description of the invention, the embodiments and to the several drawings herein.

### BRIEF DESCRIPTION OF THE DRAWINGS

The exemplary embodiment(s) of the present invention will be understood more fully from the detailed description given below and from the accompanying drawings of various embodiments of the invention, which, however, should not be taken to limit the invention to the specific embodiments, but are for explanation and understanding only.

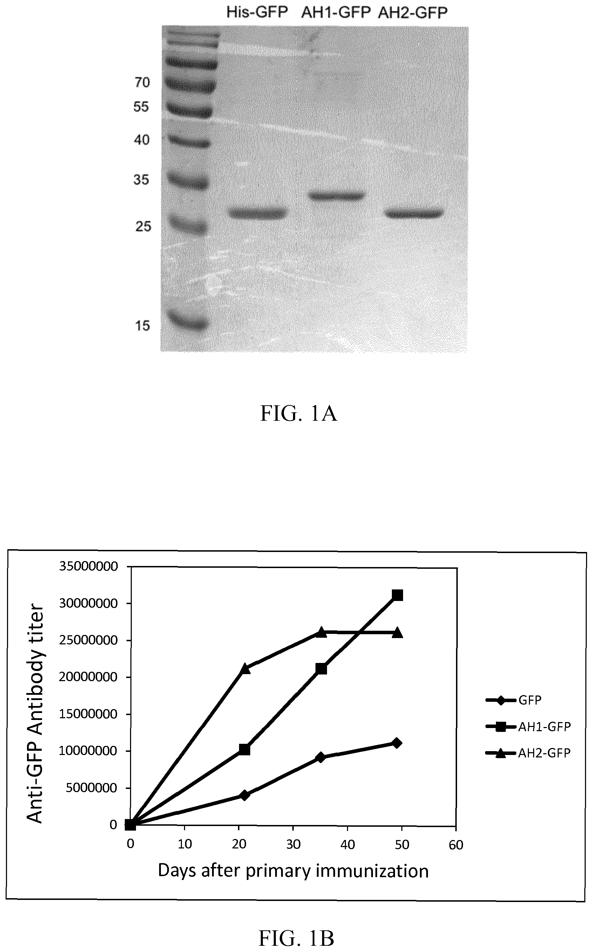

FIG. 1A shows a SDS-PAGE and coomassie blue staining result of purified His-GFP, AH1-GFP and AH2-GFP proteins.
FIG. 1B shows the result of the anti-GFP IgG titer of serum preparations from mice immunized with His-GFP, AH1-GFP or AH2-GFP as determined by ELISA. 50 µg of either His-GFP, AH1-GFP or AH2-GFP fusion proteins was used to immunized mice through peritoneal injection after emulsification with complete Freund's adjuvant (day 0) or incomplete Freund's adjuvant (day 14, 28 and 42). Serum was collected 7 days post booster doses (day 21, 35 and 49) (N=5).

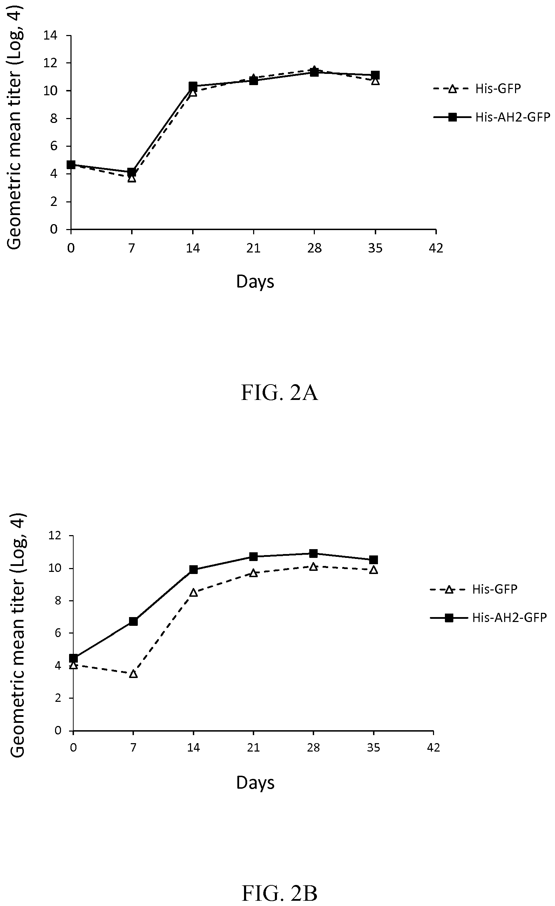

FIG. 2A shows the result of the anti-GFP IgG titer of serum preparations from mice immunized with 20 µg each of His-GFP or AH2-GFP proteins emulsified in complete Freund's adjuvant. These mice were immunized through peritoneal injection at day 0 and serum collected at day 7, 14, 21, 28 and 35 for ELISA. Geometric mean titer is shown as Log4 (N=5).
FIG. 2B shows the result of anti-GFP IgG titer of serum preparations from mice immunized with 20 µg each of His-GFP or AH2-GFP proteins emulsified in incomplete Freund's adjuvant. These mice were immunized through peritoneal injection at day 0 and serum collected at day 7, 14, 21, 28 and 35 for ELISA. Geometric mean titer is shown as Log4 (N=5).

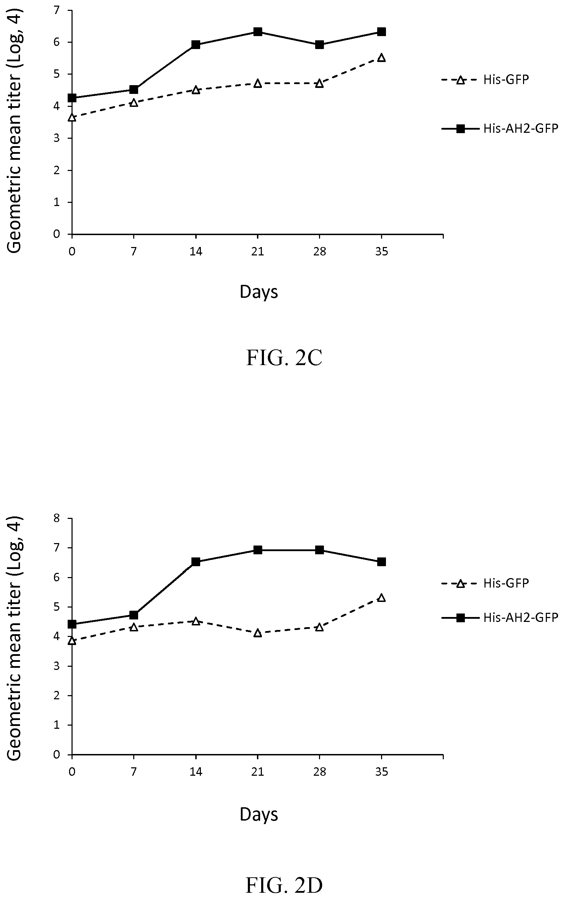

FIG. 2C shows the result of the anti-GFP IgG titer of serum preparations from mice immunized with 20 µg each of His-GFP or AH2-GFP proteins together with 10 µg of TLR5 ligand, flagellin. These mice were immunized through peritoneal injection at day 0 and serum collected at day 7, 14, 21, 28 and 35 for ELISA. Geometric mean titer is shown as Log4 (N=5).
FIG. 2D shows the result of the anti-GFP IgG titer of serum preparations from mice immunized with 20 µg each of His-GFP or AH2-GFP proteins alone. These mice were immunized through peritoneal injection at day 0 and serum collected at day 7, 14, 21, 28 and 35 for ELISA. Geometric mean titer is shown as Log4 (N=5).

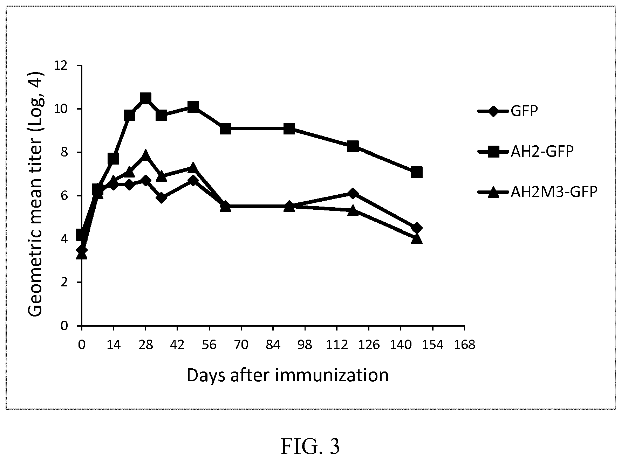

FIG. 3 shows the result of the anti-GFP IgG titer of serum preparations from mice immunized with 20 µg each of His-GFP, AH2-GFP or AH2M3-GFP proteins. Mice were immunized twice at day 0 and day 14 by intramuscular injection. Geometric mean titer is shown as Log4 (N = 5).

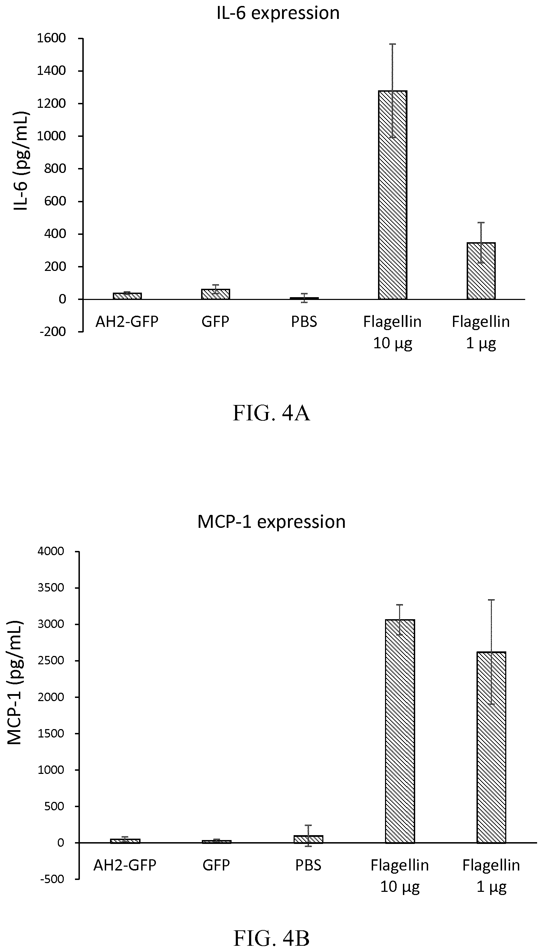

FIG. 4A shows the result of determination of plasma IL-6 level in mice. Mice were injected with 50 µg AH2-GFP, 50 µg GFP, 20 µl PBS, 10 µg flagellin or 1 µg flagellin through an intramuscular injection. Two hours post injection, plasma was prepared from blood withdrawn through cardiac puncture under anesthesia. The plasma IL-6 level was determined by Sandwich ELISA quantified by comparing to standard curve (N = 5).
FIG. 4B shows the result of determination of plasma MCP-1 level in mice. Mice were injected with 50 µg AH2-GFP, 50 µg GFP, 20 µl PBS, 10 µg flagellin or 1 µg flagellin through an intramuscular injection. Two hours post injection, plasma was prepared from blood withdrawn through cardiac puncture under anesthesia. The plasma MCP-1 level were determined by Sandwich ELISA and quantified by comparing to standard curve (N=5).

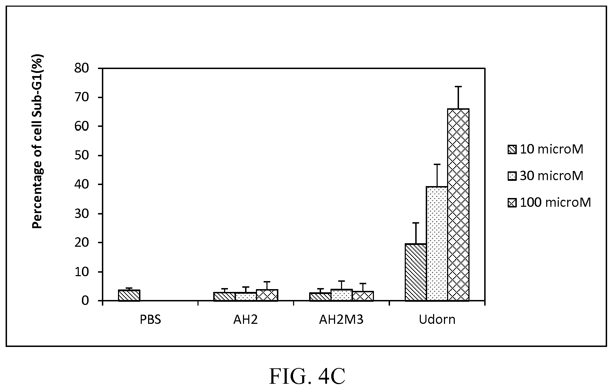

FIG. 4C shows the result of determining the effect of amphipathic helical peptide on cell apoptosis. MDCK cells grown on 6-well plates were treated with 10 µM, 30 µM or 100 µM concentrations of AH2, AH2M3 or Udorn peptides for 24 hours before cell harvest and incubation with SYTOX AADvanced Dead Cell Stain and analyzed with a flow cytometer. Sub-G1 fractions were calculated and averaged from 3 samples in each experimental group.

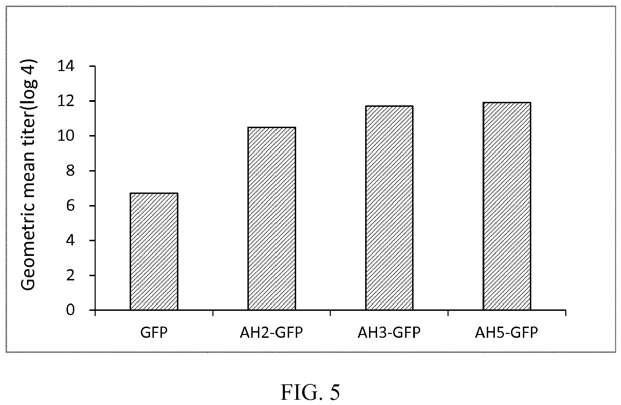

FIG. 5 shows the result of the anti-GFP IgG titer of serums from mice immunized with GFP, AH2-GFP, AH3-GFP or AH5-GFP fusion proteins. The mice were immunized with two intramuscular injections of 20 g each of various proteins with 2 weeks interval. The anti-GFP IgG titer at day 28 was used for comparison. Geometric mean titer is shown as Log4 (N=5).

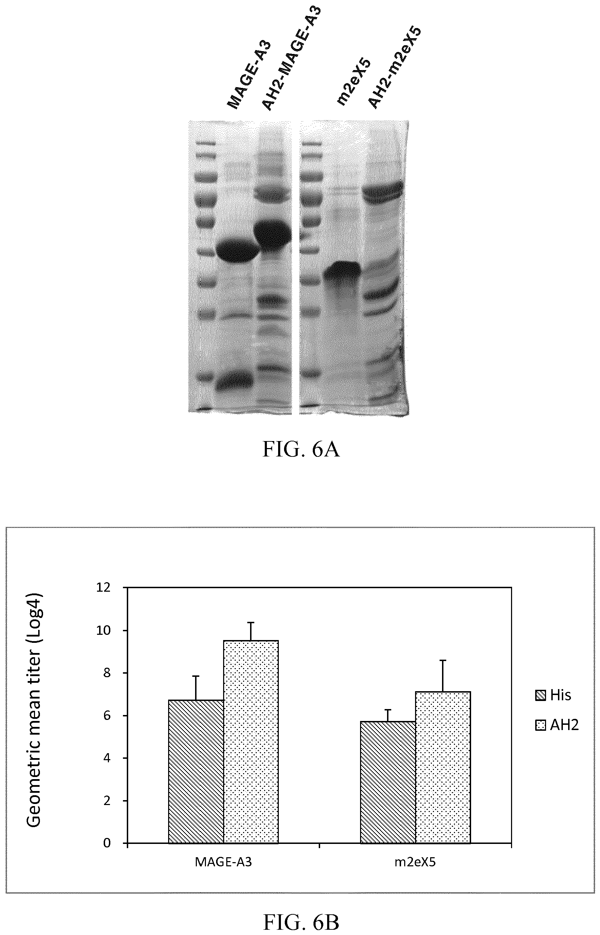

FIG. 6A shows the result of SDS-PAGE and coomassie blue staining of purified MAGE-A3, AH2-MAGE-A3, m2eX5 and AH2-m2eX5.
FIG. 6B shows the stimulation of anti-MAGE-A3 and anti-m2eX5 IgG titer by adding AH2. The mice were immunized with 20 µg of MAGE-A3, 20 µg of AH2-MAGE-A3, 8 µg of m2eX5 or 8 µg of AH2-m2eX5 fusion proteins by intramuscular injection at day 0 and day 14. The serum was collected at day 28 to measure the IgG titer against either MAGE-A3 or m2eX5 protein. Geometric mean titer is shown as Log4 (N = 5).

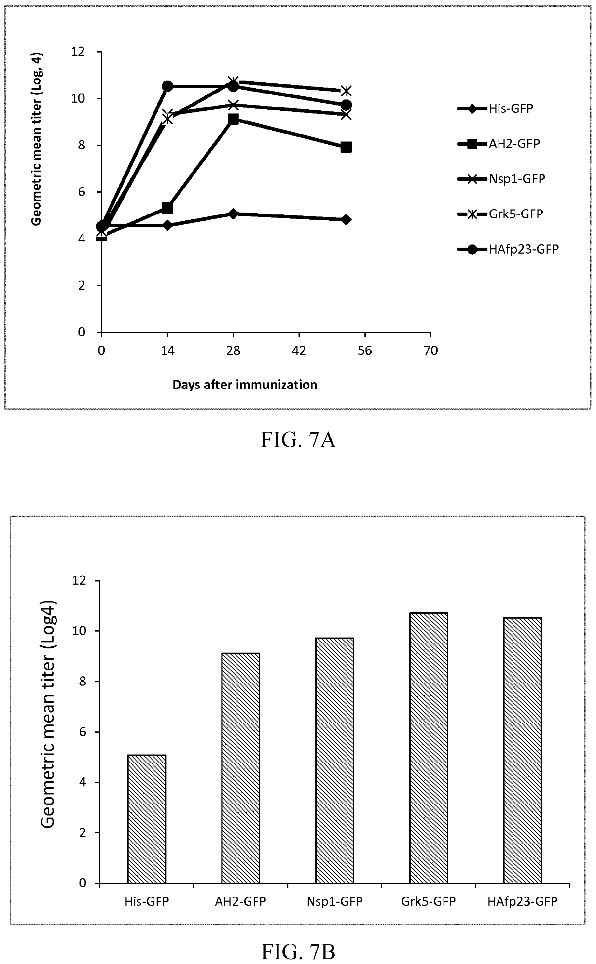

FIG. 7A shows the line chart of anti-GFP IgG titer. The mice were immunized with an intramuscular injection of 20 µg of each His-GFP, AH2-GFP, NSP1-GFP, GRK5-GFP, HAfp23-GFP and AH1-GFP at day 0 and day 14, respectively. The serum collected at day 0, 14 and 28 were used for ELISA to detect anti-GFP IgG titer. Geometric mean titer is shown as Log4 (N=5).
FIG. 7B shows the bar graph of anti-GFP IgG titer at day 28 after primary immunization. The mice were immunized with an intramuscular injection of 20 µg each of His-GFP, AH2-GFP, NSP1-GFP, GRK5-GFP and HAfp23-GFP at day 0 and day 14. Anti-GFP IgG titers of day 28 serum are used for comparison. Geometric mean titer is shown as Log4 (N=5).

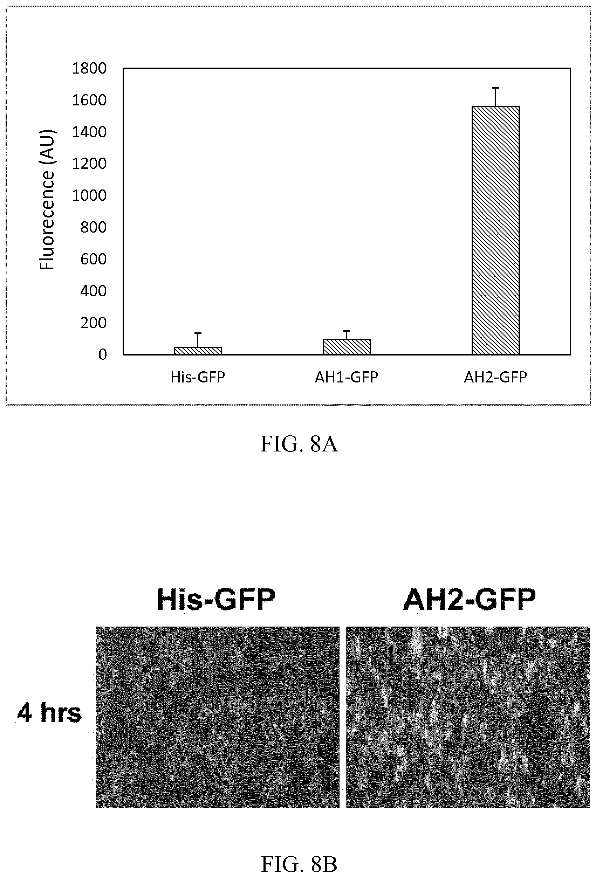

FIG. 8A shows the result of cell binding and internalization of GFP proteins fused with AH2 peptide. Purified His-GFP, AH1-GFP and AH2-GFP fusion proteins were incubated with cultured MDCK cells in a 96-wells plate at a concentration of 200 µg/ml for 4 hours before washing with 200 µl PBS twice. The amounts of GFP fusion proteins attached to the cells were then determined with a fluorometer. The fluorescence levels from 4 separate wells were used for quantification and statistical analysis.
FIG. 8B shows the result of detection of the GFP fusion protein with a fluorescent microscope. Cells incubated with the GFP fusion protein for 4 hours were washed and used for detecting the GFP fusion protein under a fluorescent microscope. The image obtained from GFP channel was merged with a DIC image in the same field to show the location of GFP signal.

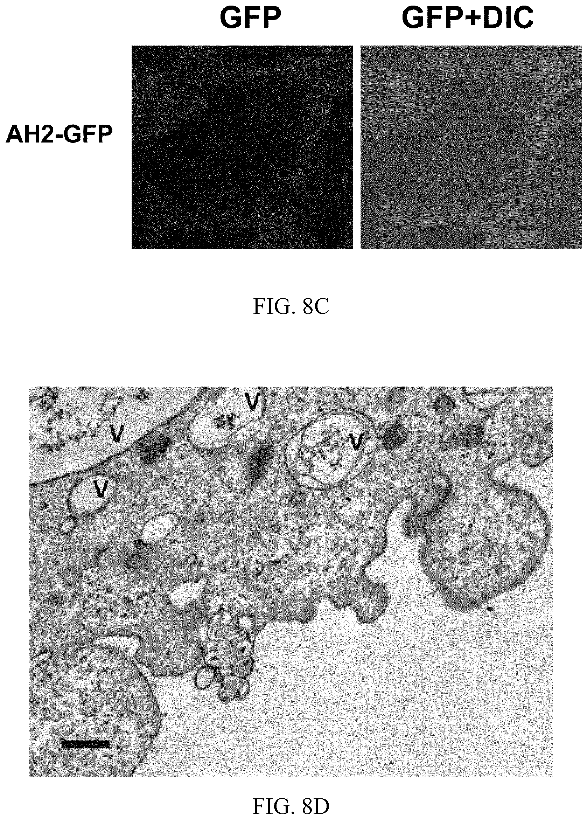

FIG. 8C shows the detection result of internalized GFP fusion proteins using a confocal microscope. AH2-GFP protein was incubated with cultured MDCK cells for 4 hours at 200 µg/ml concentration. The subcellular localization of AH2-GFP fusion proteins were then detected using a confocal microscope.
FIG. 8D shows the identification result of internalized AH2 peptides using a transmission electronic microscope. Gold nanoparticles 10 nm in size and conjugated with AH2 peptide were mixed with 10 µM of AH2 peptide and incubated with cultured MDCK cells for 4 hours before fixation and processed for observation under TEM. The vesicles that formed by AH2 peptide were marked by "V". The size bar is 0.5 µm.

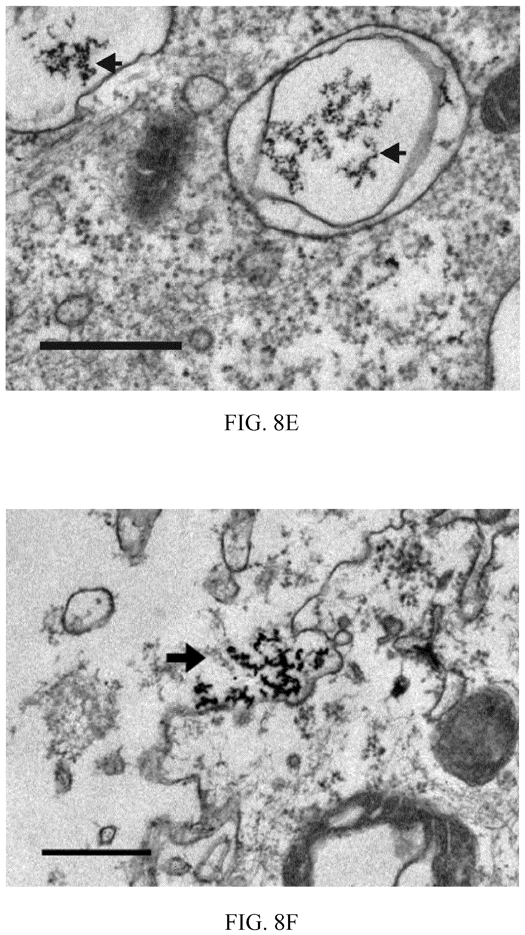

FIG. 8E shows the identification result of AH2 peptide/lipid complex with a TEM. Under TEM, there is a mesh-like structure inside the vesicle formed by AH2 peptide as indicated by arrow. The size bar is 0.5 µm.
FIG. 8F shows that the arrow points to the mesh-like structure that is formed by the AH2 peptide, AH2 peptide conjugated gold nanoparticles and the membrane was secreted out from the cells. The size bar is 0.5 µm.

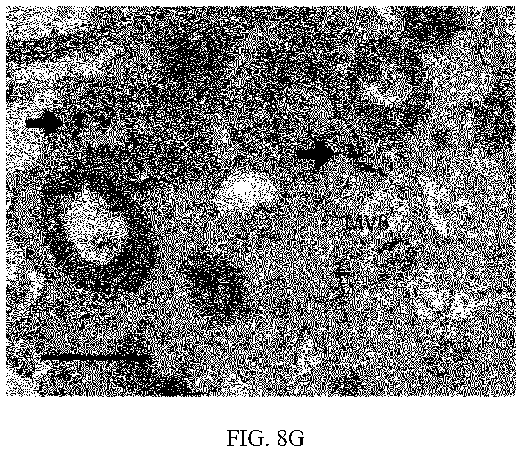

FIG. 8G shows that the arrow points to the mesh-like structure that is formed by the AH2 peptide, AH2 peptide conjugated gold nanoparticles and the membrane was enclosed in a multiple vesicle body (MVB) structure, part of endosome system. The size bar is 0.5 µm.

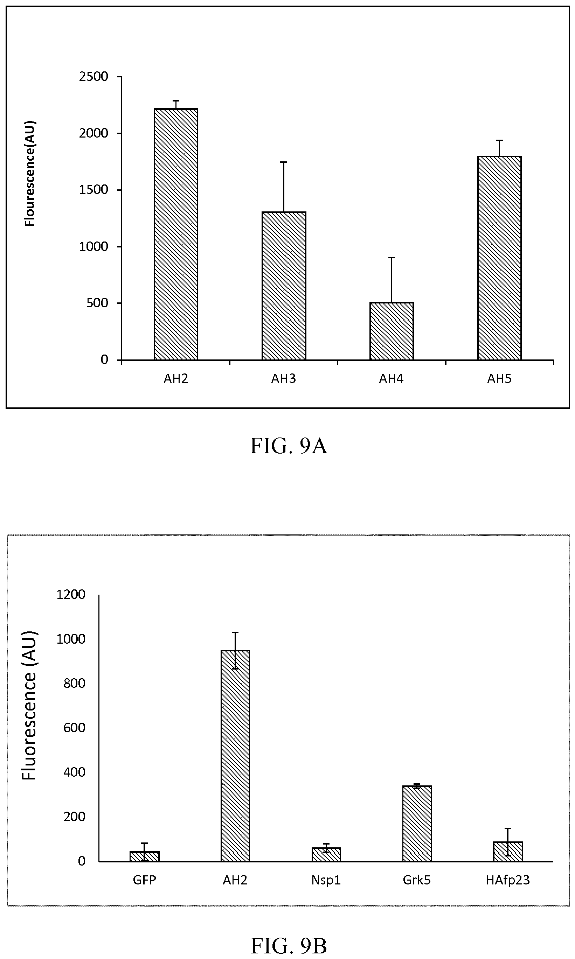

FIG. 9A shows the result of cell binding and internalization assay of synthesized amphipathic peptides labeled with FITC. Peptides corresponding to amphipathic helix of M2 protein from different Type A Influenza virus strains were synthesized and used to incubate with cultured MDCK cells for 4 hours before washing and detection in a fluorometer. SEQ ID NO: 4, is from H1N1. SEQ ID NO: 5 is from H5N1. AH4, DRLFFKCVYRLFKHGLKRG, is from H3N2. SEQ ID NO: 6 is from H7N9. Each data is quantified from 4 repetitive samples (N=4).
FIG. 9B shows the result of the cell binding assay of GFP fusion proteins. Amphipathic helical peptides derived from amino acid residue 245-264 of Nsp1 protein from Semliki Forest virus, Nsp1; amino acid residue 546-565 of G-protein coupled protein kinase 5, Grk5; fusion hairpin peptide of hemagglutinin, HAfp23, were fused to GFP in a pET28a vector using DNA recombination techniques. GFP fusion proteins were expressed and purified and used to incubate with cultured MDCK cells for 4 hours before washing and detection in a fluorometer. Each data is quantified from 4 repetitive samples (N=4).

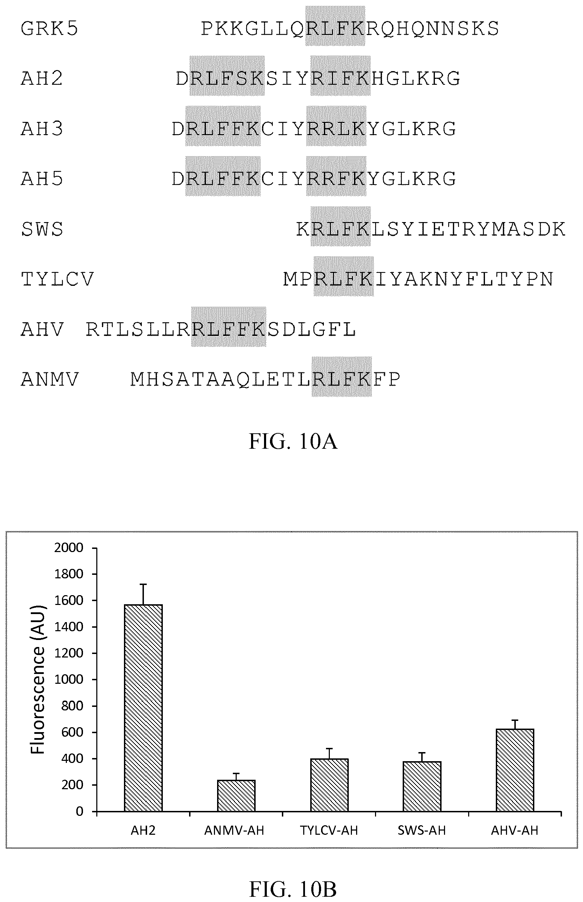

FIG. 10A shows the alignment of peptide sequences that might provide cell binding and internalization activities. Peptide sequences from Grk5, AH2, AH3, AH5, SWS-AH, TYLCV-AH, ANMV-AH, and AHV-AH are aligned to show the RLFK/RLFFK motif.
FIG. 10B shows the result of the transport of synthesized peptides corresponding to AH2, ANMV-AH, TYLCV-AH, SWS-AH and AHV-AH sequences were used for the cell binding and internalization assay. All peptides are N-terminal labeled with a FITC fluorophore. MDCK cells were incubated with 10 µM each of the tested peptides for 4 hours before PBS washing twice and fluorescence detection by a fluorometer. Background fluorescence was subtracted using a PBS negative control.

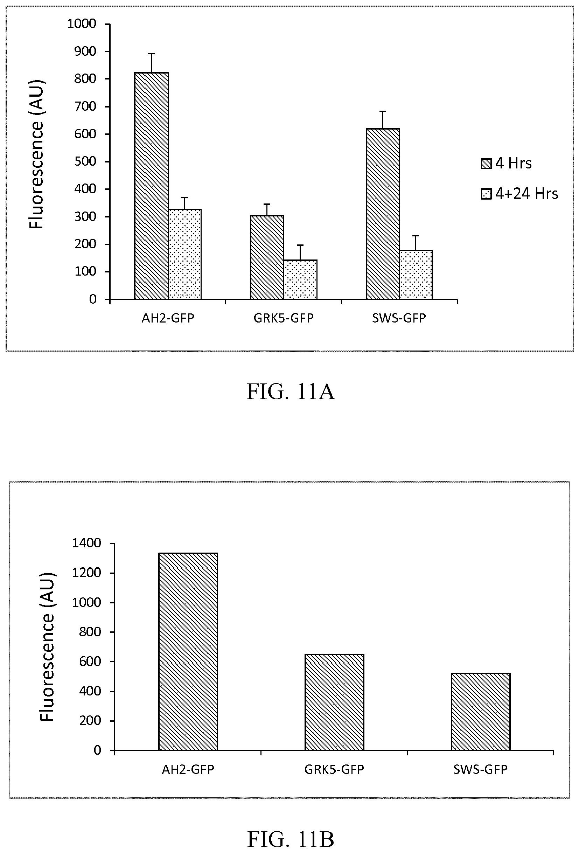

FIG. 11A shows that the decay of fluorescence level from cell loaded with GFP fusion proteins. MDCK cells were incubated with AH2-GFP, GRK5-GFP or SWS-GFP fusion protein for 4 hour before being washed and digested with 0.4U thrombin for 20 minutes. After thrombin treatment, the cells were washed with PBS and either detected with a fluorometer (4 Hrs) or incubation was continued for another 24 hours before washing and detection (4+24 Hrs) (N=4).
FIG. 11B shows the detection of secreted GFP fusion protein in the media. Media removed from cells cultured in the 4+24 hours group were used for measuring fluorescence level in a fluorometer. Media from a parallel PBS control were used to determine background fluorescence. Background fluorescence was subtracted from each group.

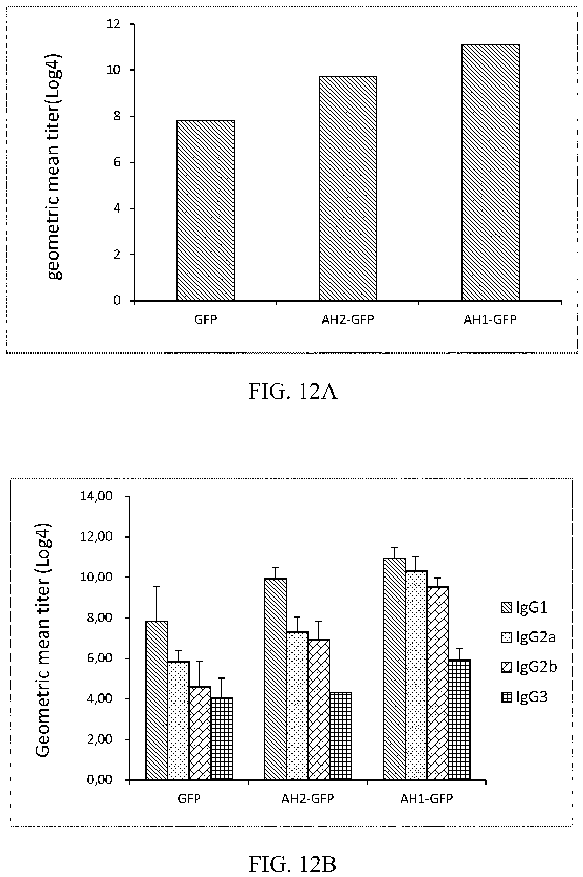

FIG. 12A shows the determination of anti-GFP IgG titer. BALB/c mice of 8 weeks old were immunized with an intramuscular injection of 20 µg each of His-GFP, AH2-GFP or AH1-GFP at day 0 and day 14. Sera were collected at 28 days after primary immunization for determining the total anti-GFP IgG titer (N=5).
FIG. 12B shows the determination of anti-GFP IgG subclass titer. BALB/c mice of 8 weeks old were immunized with an intramuscular injection of 20 µg each of His-GFP, AH2-GFP or AH1-GFP at day 0 and day 14. Sera were collected at 28 days after primary immunization for determining the IgG subclasses titer against GFP. HRP conjugated goat anti-mouse IgG1, goat anti-mouse IgG2a, goat anti-mouse IgG2b and goat anti-mouse IgG3 secondary antibodies were used in the ELISA assay.

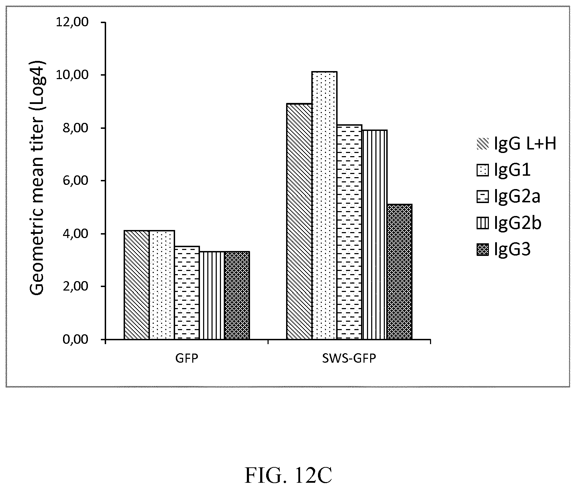

FIG. 12C shows the determination of anti-GFP IgG subclass titer induced by SWS-GFP fusion protein. BALB/c mice were immunized with an intramuscular injection of 20 µg His-GFP or SWS-GFP at a hind limb thigh. Sera collected at day 14 post immunization were used for determining anti-GFP IgG or IgG subclasses by ELISA. The result is shown as Log4 (N=5).

### DETAILED DESCRIPTION OF THE PREFERRED EMBODIMENTS

Exemplary embodiments of the present invention are described herein in the context of a method of improving immune response by using an antigen-complex with amphipathic helix structure.

Those of ordinary skill in the art will realize that the following detailed description of the exemplary embodiment(s) is illustrative only and is not intended to be in any way limiting. Other embodiments will readily suggest themselves to those skilled in the art having the benefit of this disclosure. Reference will now be made in detail to implementations of the exemplary embodiment(s) as illustrated in the accompanying drawings. The same reference indicators will be used throughout the drawings and the following detailed description to refer to the same or like parts.

As used herein, the articles "a" and "an" refer to one or more than one (*i*.*e*., at least one) of the grammatical object of the article. By way of example, "an element" means one element or more than one element.

The term "subject" can refer to a vertebrate having immune response or to a vertebrate deemed to be in need of immune response. Subjects include all warm-blooded animals, such as mammals, such as a primate. The term subject includes domesticated animals, such as cats, dogs, etc., livestock (for example, cattle, horses, pigs, sheep, goats, etc.) and laboratory animals (for example, mice, rabbits, rats, gerbils, guinea pigs, etc.). Thus, veterinary uses and medical formulations are contemplated herein.

As used herein, "antigen-complex" refers to a recombinant protein composed of various amphipathic peptides and exogenous molecules that are biocompatible with hosts, such as the green fluorescent protein (GFP). The exogenous molecules may be selected from the group consisting of, for example, but not limited to, oligosaccharide, polypeptide, protein, polysaccharide, peptide and a combination thereof.

The terms "amphipathic helical structure", "amphipathic peptide", "amphipathic molecule" or "amphipathic helix" used in the present invention indicate that a molecule, especially a peptide consisting of amino acids, forms an α helix and possesses both hydrophobic and hydrophilic elements. The amphipathic peptide comprises the sequence of amino acids obtained from, for example but not limited to, the genome of viruses, the short segment from protein-coupled receptor kinases and fusion peptide domains.

In related embodiments, the amphipathic peptides include, for example, the genome of viruses, the short segment from protein-coupled receptor kinases and fusion peptide domains. The exemplary amphipathic peptides are shown in Table 1.

**Table 1, SEQ ID NO: 1 - SEQ ID NO: 11**

| **SEQ ID No.** | **Sequence** | **Source** |
|---|---|---|
| 1 | Amino Acid Sequence: TRGLLGCIITSLTGR | Hepatitis C virus isolate H77, NS3 protein |
| 2 | Amino Acid Sequence: GSTLYTESRKLLRSWHLPSV | Semliki Forest virus replicase nsP1 protein |
| 3 | Amino Acid Sequence: GLFGAIAGFIEGGWTGMIDGWYG | fusion hairpin peptide of hemagglutinin |
| 4 | Amino Acid Sequence: DRLFSKSIYRIFKHGLKRG | Influenza A virus (A/TW/3355/97(H1N1)) M2 (M2) and M1 (M1) genes, complete cds |
| 5 | Amino Acid Sequence: DRLFFKCIYRRLKYGLKRG | Influenza A virus, H5N1 |
| 6 | Amino Acid Sequence: DRLFFKCIYRRFKYGLKRG | Influenza A virus, H7N9 |
| 7 | Amino Acid Sequence: PKKGLLQRLFKRQHQNNSKS | Amino acid residues 546-565 of G-protein coupled receptor kinase 5 |
| 8 | Amino Acid Sequence: KRLFKLSYIETRYMASDK | Amino acid residues 8-25 of WSV445 protein from Shrimp white spot syndrome virus |
| 9 | Amino Acid Sequence: MPRLFKIYAKNYFLTYPN | Amino acid residues 1-18 of replication-associated protein from Tomato |
| | | yellow leaf curl virus. |
| 10 | Amino Acid Sequence: RTLSLLRRLFFKSDLGFL | Amino acid residues 33-50 of ORF 48 from Ateline herpesvirus 3. |
| 11 | Amino Acid Sequence: MHSATAAQLETLRLFKFP | Amino acid residues 17-34 of a hypothetical protein (gb\|AJP62030.1) from ANMV-1 virus. |

### Materials and Methods

First, cloning technology was used to obtain fusion proteins. Then, after purification of the proteins, endotoxins are removed using polymyxin resin to obtain a protein preparation with an endotoxin level less than 5 EU/mg. These purified proteins were injected into mice to induce immunization. Further, mice sera were isolated for antibody titer determination by ELISA at 7, 14, 21, 28 and 35 days post protein injection. Steps of the detailed protocols are described as follows.

### Expression and purification of endotoxin free protein

### Protein expression

The pET28a vector encoding target protein was transformed into BL21 (DE3) using heat shock. The procedure for heat shock transformation is as follow.

Day 1 at 3pm, plasmid of 1ng was mixed with 50 µl thawed competent cell, BL21(DE3), and incubated on ice for 10 minutes before applying 42°C heat shock for 40 seconds. After heat shock, mixture was kept on ice for 1 minute before adding 1ml LB and then shook at 220 rpm at 37°C for 1 hour. Then bacteria were spun down at 13000 rpm for 1 minute. The bacteria pellet was re-suspended in 100 µl LB and plated on LB plate with 100 µg/ml ampicillin and incubated at 37°C.

Day 2, at 10 am, there should be less than 100 colonies on the plate. One colony was picked and inoculated with 3ml LB/ampicillin culture and incubated at 37°C/225 rpm. By 12 am, the 3ml culture was transferred to 500ml LB/ampicillin media and the growth of culture monitored by spectrophotometer. When OD600 reached 0.5-0.7, 1mM IPTG was added into the culture to induce protein expression. The culture was harvested 3 hours after protein induction. Bacteria were spun down by centrifuge at 5000 rpm for 10 minutes. Supernatant were removed and the pellet kept for further procession.

### Protein purification

Buffer for protein purification and gel filtration: 20mM NaPO₄, pH 7.4, 300mM NaCl (GF buffer). For Ni-NTA resin purification: 5mM Imidazole in GF for bacteria lysis (Lysis buffer), 10mM Imidazole in GF for wash (Wash buffer), 200mM Imidazole in GF for protein elution (Elution buffer). Bacteria pellet from 500ml LB culture was re-suspended in 40 ml Lysis buffer by vortex and then lysed using ultrasonic sonicator (Misonix 3000) at 10S on/5S off cycles for 5 minutes. The re-suspended bacteria were kept in icy water during sonication. Insoluble debris was removed by centrifugation in 10000 rpm for 10 minutes using Sorvall SS34 rotor at 4°C. Supernatant containing target protein was passed through a polypropylene column contained 2ml Ni-NTA resin from Qiagen. After the target protein has bound to the Ni-NTA resin, the column was washed by 10 times with a bed volume of wash buffer (20 ml). After washing, the target protein was then eluted using 5ml elution buffer. Target protein purified using Ni-NTA resin was further purified using Gel filtration column from GE (HiLoad 16/600 Superdex 200 pg) on AKTA prime.

### Gel filtration

Three programs were setup for the purification of proteins in the study. Program 30: For gel filtration purification of target protein. Program 33: for onsite cleaning of HiLoad 16/600 superdex 200 pg using 120ml 0.5M NaOH. Flow rate is 0.8ml/minutes. Program 34: for equilibrate column after onsite cleaning using 150ml GF buffer. Column was washed after two protein purifications. A 5ml sample loop was connected to the machine, AKTA prime, for holding protein sample. Sample loop is first flushed using 25 ml GF buffer to remove residual protein in previous purification. Before loading target protein into the sample loop, additional 0.5ml GF buffer was added into the Ni-NTA purified protein. Protein solution was placed into a 5 ml syringe and loaded into the sample loop. Glass tubes in the corresponding fractions of target protein eluted were replaced with new tubes every time before the purification. All the buffers to be used in the gel filtration experiment were filtered through a 0.2 µm filter unit for sterilization and removal of impurity. These buffers were further degassed by vacuum for 10 minutes. FPLC Fractions contained target proteins as monitored by UV280 were collected and pooled together. The pooled protein solution was concentrated by 2^{nd} Ni-NTA column with same bed volume as first one. After the protein was eluted from 2^{nd} Ni-NTA column, protein concentration was determined by BCA method. Two fractions with highest protein concentration were pooled and dialyzed against 1L PBS overnight. After dialysis, endotoxin contaminant was removed using EndotoxinOUT^{™} resin from G-Bioscience. The polypropylene column was filled with 4ml EndotoxinOUT^{™} resin and washed with 5 bed volume of 1% sodium deoxycholate in LAL Reagent Water (LRW), 5 bed volume of LRW and 5 bed volume of PBS/LRW before loading 1.5ml target protein. After complete loading of the protein, column was placed in cold room for 1 hour for complete absorption of endotoxin by resin. Protein was eluted using step wise addition of first fraction 0.5 ml and later fractions 1 ml of PBS. Protein was eluted in the 3^{rd} to 6^{th} fractions.

### LAL (limulus amoebocyte lysate) assay

Endotoxin level was determined using PYROTELL-T LAL provided by Associate of Cape Cod, Inc. Each LAL reagent supplied in lyophilized powder is first dissolved in 5ml LRW and then aliquot into small volume and kept in -80 °C. In a typical turbidity assay using PYROTELL-T, 100 µl of sample including endotoxin standard were applied in a microplate. The reaction kinetic was measured using a VICTOR3 fluorescence reader by OD405 at 37°C. The time-lapse between each reading was 70 seconds and the reading was repeated for 60 times. The time needed for a reaction to reach a difference of 0.1 in OD405 between sample and blank was recorded and compared to the endotoxin standard to determine the endotoxin unit in a sample. The log of endotoxin activity was plotted against time (seconds) to reach a difference of 0.1 in OD405; a logarithmic trend line is used as standard curve. The identified endotoxin activity in a protein sample was further combined with protein concentration, as determined by BCA assay, to determine specific endotoxin activity, EU/mg. The upper limit of specific endotoxin activity in all the protein samples was 5 EU/mg.

### Immunization of mice in the absence of adjuvant

Mice used in immunization procedures are all between 6 and 8 weeks of age. Without anesthetization, mice were bled from the tail by clipping away 2-3 mm of tail. Blood was collected using pipetman with occasional massaging on the tail. About 100 µl blood was collected from each bleeding. Blood was left on RT for 2 hours before centrifugation at 2000 rpm for 10 minutes in a microcentrifuge, and serum was collected in a microtube. Remained red blood cells were removed using 2000 rpm for 10 minutes in a microcentrifuge. Serum was stored in -80 °C before use in an ELISA assay. Mouse tail bleeding is stopped with a heated soldering iron and pressing the tail tip between thumb and index finger. For primary immunization, 20 µg purified protein was intramuscularly injected in the thigh of a hind limb. Blood was drawn on day 14 post immunization from the tail and a booster dose with the same amount of purified protein was injected with the same procedure.

### ELISA for determining antibody titer

Dilute the GFP protein with coating buffer and coat appropriate wells of ELISA plate with the GFP at a concentration of 10 µg/ml. Cover the plate with an adhesive plastic and incubate at 4°C overnight. Wash the plate three times with 300 µl of washing buffer. Add 200 µl of blocking buffer to block the non-specific binding sites in the coated wells. Cover the plate with adhesive plastic and incubate at 37 °C for 1 hour. Wash the plate three times with 300 µl of washing buffer. Dilute the primary antibody or antiserum with blocking buffer and add 100 µl of the diluted antibody to each well of the plate. Cover the plate with an adhesive plastic and incubate at 37 °C for 1 hour. Wash the plate three times with 300 µl of washing buffer. Dilute the HRP-conjugated secondary antibody with blocking buffer and add 100 µl of the diluted secondary antibody to each well of the plate. Cover the plate with an adhesive plastic and incubate at 37 °C for 30 minutes. Wash the plate five times with 300 µl of washing buffer. Add 100 µl of the TMB reagent per well with a multichannel pipette. After sufficient color development, add 100 µl of stop buffer to the wells. Read the absorbance of each well using 450 nm. When measuring antibody titer against MAGE-A3 and m2eX5, MAGE-A3 and m2eX5 with his tag were used to coat the plate at 10 µg/ml concentration. Coating buffer contains 0.2M Carbonate/Bicarbonate buffer at pH 9.4. Washing buffer contains 0.05% Tween 20 in 1X PBS. Blocking buffer contains 1g BSA in 100 ml washing buffer. Stop buffer contains 2M sulfuric acid.

### Fluorescent labeled peptide and GFP fusion protein internalization and recycle assay

The GFP fusion protein internalization activity was tested by first plating 4x10⁴ MDCK cells on one well of a 96 well plate, each sample was tested in quadruplicate. On second day, 200 µg/ml of GFP fusion protein was added into the culture media and incubated for 4 hours. After incubation, media was removed and washed with 200µl of PBS for three times. The amount of GFP fusion protein internalized was determined by a fluorometer. For fluorescence labeled peptide, the concentration of peptide in internalization assay is 10 µM. To evaluate the efficiency of GFP fusion protein to recycle back to media, cells were first incubated with GFP fusion protein as described above. After washing with PBS, 100 µl of PBS with 0.4 unit of thrombin was used to cleave GFP from amphipathic peptide bound on extracellular surface of plasma membrane. The thrombin digestion was carried out in 37 °C incubator for 20 minutes. Then cells were washed three times with 200 µl PBS again and then incubated for 24 hours in fresh culture media. The recycling back to culture media of the GFP fusion protein was determined by removing 80 µl of media for detection of fluorescence level and using a PBS control set up in parallel to calibrate the background fluorescence.

### Plasma cytokine level determination

To detect blood cytokine level, mice were first intramuscularly injected with experimental proteins. Two hours after injection, the mice were anaesthetized before collecting blood through a cardiac puncture with a syringe preloaded with 10 µl of 0.5M Na₂EDTA. Blood collected was centrifuged at 2000 rpm for 20 minutes at a tabletop microcentrifuge. Plasma collected was aliquoted into 20 µl fractions and snap frozen on dry ice. The plasma cytokine level was determined by sandwich ELISA with a standard from eBioscience, USA. IL-6 level was detected using Mouse IL-6 ELISA Ready-SET-Go! (Cat#88-7064, eBioscience, USA). MCP-1 was detected using Mouse CCL2 (MCP-1) ELISA Ready-SET-Go! Kit (Cat#88-7391, eBioscience, USA). The detecting procedures followed the manufacturer's standard protocol.

### Cell apoptosis assay by flow cytometer

MDCK cells were first plated on 6 well plates at 3x10E5 cell/well. 24 hours after plating, peptides were applied at 10 µM concentration and incubated for 24 hours before cell harvest by trypsin digestion. The suspended cells due to apoptosis were collected by centrifugation of the culture media at 1000 rpm for 5 minutes. The cells collected from trypsin digestion and cultured media were combined and 1x10⁵ cells were fixed in 3ml 70% ethanol overnight in a -20 °C freezer. After fixation, cells were washed and resuspended in 1 ml PBS and stained by SYTOX AADvanced Dead Cell Stain kit (Thermo Fisher Scientific Inc.) in the presence of 100 µg RNase A for 30 minutes in 4 °C cold room before analysis with a flow cytometer (BD FACSCalibur™). The sub-G1 fraction was calculated and designated as apoptotic cells.

### Transmission electronic microscope

MDCK cells were seeded on ACLAR Embedding film in a 6 well plate at density of 3x10⁵ cell per well. After overnight culturing, part of the media were removed to leave 0.5ml of culture media before adding AH2 peptide to 10 µM and 50 µl of 10 nm gold nanoparticle conjugated with AH2 peptide. The concentration of AH2 conjugated 10 nm gold nanoparticle used was 4.78x10¹²/ml. After 4 hours incubation, cells were washed in serum-free culture media at room temperature before fixation in 2.5% glutaraldehyde/0.1% tannic acid/0.1M cacodylate buffer, pH 7.2 at room temperature for 30 minutes. Then cells were washed in 0.1M cacodylate buffer pH 7.2/0.2M sucrose/0.1% CaCl₂ for 5 minutes at room temperature. Afterward, cells were post-fixed in 1% OsO₄/0.1M cacodylate buffer, pH 7.2 at room temperature for 30 minutes. Then cells were washed three times with ddH₂O for 5 minutes each at room temperature. Cells were further stained with 1% uranyl acetate at room temperature for 30 minutes before being washed 3 times with ddH₂O. The cells were then dehydrated and embedded in EPON by polymerized at 60 degree for 24 hours. Embedded cells were then sectioned for observation under transmission electronic microscope.

### Example 1

Amphipathic helix peptides stimulate humoral immunity against fusion antigen. To evaluate the effect of amphipathic helix in stimulating humoral immunity against antigen it fused, amphipathic helix peptide from HCV NS3 (AH1) and Type A Influenza virus strain H1N1 M2 protein (AH2) were chosen based on the high solubility of their fusion proteins. Oligonucleotides were synthesized and used in a PCR reaction to generate the coding sequences of these two peptides and cloned into a pET28a vector containing a green fluorescent protein, GFP. Amphipathic helix peptide and GFP fusion proteins were expressed using a bacterial expression system and purified using Ni-NTA agarose. Proteins eluted from Ni-NTA resin were further purified using gel filtration in a HiLoad 16/600 superdex 200 column. Protein eluted from gel filtration column is concentrated by an Amicon Ultra 15 with a MWCO of 10K. The endotoxin contaminating in the protein preparation was removed using EndotoxinOUT^{™} resin from G-biosciences.

The ability of amphipathic helix peptides in stimulating antibody production in an adjuvant reaction is first tested in a standard Complete-incomplete Freund's adjuvant paradigm. During primary immunization, proteins in the concentration of 1 mg/ml were mixed with complete Freund's adjuvant in a 1:1 volume ratio until emulsified. 50 µg of protein was peritoneally injected into the abdomen of each C57BL/6 mouse, 5 mice for each protein. The booster doses were administered at day 14, 28 and 42 by the same route with protein mixed with incomplete Freund's adjuvant in a 1:1 volume ratio. The result is shown in FIG. 1B, where both AH1-GFP and AH2-GFP stimulate more anti-GFP antibody than GFP alone.

### Example 2

The AH2 peptide from influenza virus H1N1 M2 protein induce adjuvant independent antibody production enhancement. AH2-GFP was first expressed and purified to contain an endotoxin level less than 5 EU/mg. Then purified AH2-GFP protein was used to immunize mice in combination with a) complete Freund's adjuvant, b) incomplete Freund's adjuvant, c) flagellin or d) PBS. The control protein is His-GFP and is expressed and purified to contain an endotoxin level less than 5 EU/mg. 50 µg of protein in the form of emulsion or mixture was injected into one mouse with 5 mice in each group and the anti-GFP antibody level was monitored by ELISA using GFP protein coating. Serum collected at 0, 7, 14, 21, 28, 35 days post injection were used. The result suggests for proteins emulsified in complete Freund's adjuvant, there is no significant difference in the anti-GFP IgG level between AH2-GFP and His-GFP (FIG. 2A). For protein emulsified in incomplete Freund's adjuvant, the AH2-GFP induced significantly higher anti-GFP IgG level in day 7 compared to His-GFP This difference in anti-GFP IgG production level continues to the day 35 (FIG. 2B). For proteins mixed with 10 µg flagellin, the anti-GFP IgG level started to show significant difference at day 14 post injection and continue to day 35 after injection (FIG. 2C). When AH2-GFP and His-GFP were injected together with PBS, the anti-GFP IgG production started to show significant difference at day 14 post injection and continue to day 35 (FIG. 2D). These results show the addition of AH2 peptide sequence alone is sufficient to induce higher antibody production against antigen, GFP.

### Example 3

The hydrophobic residues of AH2 peptide are required for enhancing the production of anti-GFP IgG in mice. To test the effects of hydrophobic residues of AH2 peptide on the enhancement of anti-GFP IgG titer, point mutations were created that replaced the three hydrophobic amino acids 4F, 81, and 12F with alanine (AH2M3). The complete sequence of AH2M3 is as follows: DRLASKSAYRIAKHGLKRG. The resulting protein AH2M3-GFP was expressed and purified and used to immunize mice. The AH2-GFP was used as positive control. The result showed that the replacement of 3 hydrophobic amino acids with alanine significantly reduced the ability of fusion protein to boost anti-GFP IgG production (FIG. 3). So the integrity of amphipathic helix is important for AH2-GFP fusion protein stimulate humoral immunity. Also, the antibody titer stimulated by AH2 fusion lasted at least 5 months after the primary immunization, suggesting the humoral immunity activated by AH2 peptide fusion is long lasting.

### Example 4

AH2-GFP fusion protein stimulates humoral immune response independent of activating innate immunity. To test whether AH2 peptide induces humoral immune response by first activating innate immune responses, the peripheral blood inflammatory cytokine level 2 hours after intramuscular injection of AH2-GFP fusion protein was determined using ELISA. His-GFP and AH2-GFP protein preparation with endotoxin level below 5 EU/mg were injected intramuscularly into 5 mice each at an amount of 50 µg per mouse. TLR5 ligand flagellin known to induce innate immune responses was used as a positive control at 10 µg per mouse or 1 µg/mouse dose. Blood was collected through a cardiac puncture 2 hours post intramuscular injection under proper anesthesia. Syringe used for blood collection is preloaded with 10 µl of 0.5M EDTA to avoid blood coagulation. Blood collected was spun for 20 minutes at 2000 rpm in an eppendorf microcentrifuge to prepare plasma. Plasma collected was aliquoted into a 20 µl/tube and frozen on top of dry ice for storage. Plasma thawed in room temperature was diluted 12X and each plasma sample was applied in duplicate to determine the IL-6 or MCP-1 cytokine level by ELISA according to manufacturer's instructions. The ELISA kit was purchased from eBioscience, mouse IL-6 Ready-SET-Go ELISA kit (catalog number: 88-7064-86) and mouse CCL2 (MCP-1) Ready-SET-Go ELISA kit (catalog number: 88-7391-86). The results showed the flagellin injection induced high level of both IL-6 and MCP-1 at level of 1200 pg/ml and 3000 pg/ml respectively when 10 µg flagellin was injected or 380 pg/ml and 2600 pg/ml when 1µg flagellin were injected. There was low expression of both IL-6 and MCP-1 for PBS, His-GFP or AH2-GFP injection, suggesting AH2-GFP protein does not induce innate immune responses.

An M2 amphipathic helix peptide derived from a specific strain of type A H1N1 influenza virus, Udorn had been shown to induce cell apoptosis through poring plasma membrane and cause the plasma membrane to be compromised in its integrity To test if AH2 peptide may also induce cell apoptosis, three different concentrations of AH2 peptide: 10 µM, 30 µM and 100 µM were incubated with MDCK cells for 24 hours before harvesting and chromosome DNA staining to detect cell apoptosis. The cell apoptosis level is determined using flow cytometry after ethanol fixation for 24 hours and RNase A treatment, then SytoX AADV staining 1 minute before being applied in a flow cytometer (BD FACSCalibur™). The percentage of cells with sub-G1 signal was calculated and presented. The results show the induction of MDCK cell apoptosis by the addition of Udorn peptide as described in the literature. The levels of cell apoptosis induced by either AH2 or AH2M3 are similar to PBS control, suggesting the cellular apoptosis induced by AH2 or AH2M3 is consistent with background level.

### Example 5

The induction of humoral immune responses by amphipathic helix peptides from M2 protein of influenza type A strain H5N1 and H7N9. M2 amphipathic helix peptide sequence derived from type A influenza virus H5N1 and H7N9 strain were fused with GFP using DNA recombination in a pET28a vector. AH3-GFP and AH5-GFP fusion protein expression vectors were transformed into BL21(DE3) by heat shock, colonies grown on a LB/Kan (50 µg/ml) plate were picked and used for 3ml LB culture inoculation and culturing overnight. Overnight bacterial culture was further used for the inoculation of 500ml culture and incubated at 15°C with shaking at 225 rpm until OD600 reach 0.5. After adding 1mM IPTG, the bacterial culture is further incubated at same condition for overnight fusion protein induction. After induction, bacteria were harvested and resuspended in lysis buffer for sonication to break bacteria open. After sonication, bacterial lysate is centrifuged at 10000 rpm for 10 minutes using SS34 rotor from Sorvall. Supernatant is collected after centrifugation. Soluble proteins are further purified using Ni-NTA resin and total amount of protein purified was determined using BCA assay. Endotoxin contamination was removed by the use of EndoFree® resin. 20 µg of purified AH3-GFP and AH5-GFP at 1 mg/ml concentration is injected intramuscularly at the caudal of a thigh. Blood were collected from tail veins by clipping the tip of the mouse's tail. Blood collected is left on a table top for several hours for coagulation. Then blood was centrifuged at 2000 rpm for 15 minutes and serum was collected. The anti-GFP IgG titer in blood was determined by ELISA using GFP protein for coating at a concentration of 10 µg/ml. The result showed the addition of AH3 or AH5 peptide in tandem with GFP protein is able to induce the elevation of anti-GFP IgG titer even more significant than AH2 peptide (FIG. 5). Suggesting AH3 and AH5 have higher activity in inducing humoral immune response than AH2, and maybe useful to reduce the total doses of vaccine needed for inducing protective immune response. After all, these amphipathic helix peptides derived from three different type A influenza strains all stimulate the humoral immune response when fused with a protein antigen.

### Example 6

The induction of humoral immunity by the MAGE-A3 and m2eX5 protein fused with AH2 peptide. The AH2 peptide from type A influenza strain H1N1 has been shown to induce high anti-GFP IgG titer when fused to GFP. To evaluate the efficacy of the AH2 peptide in stimulating humoral immunity against various antigens, the AH2 peptide was fused with either the MAGE-A3 or the m2eX5 protein through DNA recombination techniques. AH2-MAGE-A3 and AH2-m2eX5 proteins were expressed and purified as well as their respective control proteins. Purified proteins were used to immunize C57BL/6 mice through intramuscular injection. The humoral immunity responses were monitored through the detection of anti-MAGE-A3 or anti-m2eX5 IgG titer using ELISA. The results show the addition of AH2 peptide onto either MAGE-A3 or m2eX5 protein increases the corresponding IgG titer by 50 folds and 7 folds respectively when compared to their reference proteins without AH2 peptide fusion. This result suggests the AH2 peptide and its related amphipathic helical peptides are suitable to serve as a vaccine platform to induce high level of humoral immunity against pathogenic antigens.

### Example 7

Amphipathic helix peptides from GRK5, HA and NSP-1 also stimulate humoral immunity against fused protein. To evaluate whether amphipathic helix from proteins other than M2 protein of type A influenza virus also share similar activity in inducing humoral immune response against fused protein, the inventor did a literature search to identify the peptide sequences that had been shown to interact with the membrane when fused with a fluorescent protein, GFP. Three peptide sequences were identified: amphipathic helical peptides derived from amino acid residue 245-264 of Nsp1 protein from Semliki Forest virus, Nsp1; amino acid residue 546-565 of G-protein coupled receptor kinase 5, Grk5; fusion hairpin peptide of hemagglutinin, HAfp23. These peptide sequences were fused with GFP using DNA recombination techniques and fusion proteins were expressed and purified as described above. His-GFP, AH2-GFP, NSP1-GFP, GRK5-GFP and HAfp23-GFP fusion proteins were used to immunize mice. During mice immunization, each mouse was intramuscularly injected with 20 µg of purified fusion proteins at day 0 and day 14 and serum collected at 14 days post each injection.

Of the fusion of amphipathic helix peptide from NSP1, GRK5, and HA proteins, all of them potently enhance the humoral immune response against GFP, the model protein (FIG. 7A, 7B). These results suggest that the simple attachment of a short peptide having the property of amphipathic helix can significantly increase the ability of an antigen in inducing humoral immunity.

### Example 8

AH2 is able to induce the polymerization and internalization of the fusion proteins into the cell as a membrane structure. AH1-GFP, AH2-GFP and His-GFP protein were expressed and purified using Ni-NTA agarose resin and then by Gel Filtration and then concentrated by Ni-NTA agarose resin. To test the ability of these fusion proteins in binding to the cell membrane of cultured cells, fusion proteins in the concentration of 200 µg/ml were used to incubate with cultured MDCK cells. After 4 hours of incubation, unbound GFP fusion proteins were washed away using PBS buffer. Bound GFP fusion proteins were detected by fluorometer. Cells incubated with PBS alone were used as calibration for background fluorescence. The result shows the binding of AH2-GFP to cell membrane but not AH1-GFP or His-GFP (FIG. 8A). To observe the binding of the GFP fusion protein to cultured cells, 100 µg/ml of protein was added into cultured CLI-5/pENO1 cells and incubated for 4 hrs. At the end of incubation, cells were washed twice with PBS and then used for observation under a fluorescence microscope. For cells incubated with AH2-GFP, fluorescence was observed on cells at 4 hours post incubation. There was no observation of fluorescent spots on the cell surface when His-GFP was used in incubation (FIG. 8B). Over 50% of cells have fluorescent spots after 4 hours of incubation with AH2-GFP. This result suggests the AH2-GFP fusion protein can polymerize on the cell surface when provided as a soluble protein in the media. To further identify the subcellular localization of these fluorescent spots, a confocal microscope was used. When examined under a confocal microscope, these fluorescent spots were found to be shaped like endosomal vesicles and located mainly inside the cells, but not on the plasma membrane (FIG. 8C). This result suggests the AH2-GFP fusion protein is able to internalize into the cytosol of cell and form vesicle like structures. In order to track the vesicles formed inside cultured cells, the inventor incubated synthesized peptides corresponding to the amphipathic helix sequence of AH2 with cultured cells together with gold nanoparticles conjugated with AH2 peptide for tracking vesicles formed by adding AH2 peptide. The addition of AH2 peptides induced vesicles formation, marked as V, inside the cell (FIG. 8D). There are mesh-like structures inside these vesicles with gold nanoparticles decorating along these structures, as marked by an arrow, suggesting these mesh-like structures may be constituted by phospholipids and AH2 peptides (FIG. 8E). Some of the gold nanoparticle decorated mesh-like structures can be observed in a multiple vesicle body (MVB) like structure, suggesting these structures can be incorporated into the endosomal pathway (FIG. 8G). Also these mesh-like structures can be observed in extracellular space, an indication that the vesicle containing these structures may fuse with the plasma membrane and cause secretion of these structure into cultured media (FIG. 8F).

### Example 9

The amphipathic helix peptides from 3 different types of A influenza virus strains and G-protein coupled receptor kinase 5 also mediate membrane budding and internalization. The amphipathic helix peptides from type A influenza virus have been shown to be important for the ESCRT-independent budding of viral particles. They have also been shown to mediate budding in an in vitro condition when synthesized amphipathic helix peptides from M2 proteins were mixed with membrane vesicle containing cholesterol (Rossman JS 2010). To test if M2 amphipathic helix peptides from viral strains other than H1N1 also share the similar property of binding to cultured cells, the inventor synthesized three M2 amphipathic helix peptides derived from H5N1, H3N2 and H7N9 and named them AH3, AH4 and AH5, respectively. Although these three peptides did not bind to the membrane as well as AH2, they did all bind and were internalized to the cell at different levels (FIG. 9A). This result suggests that these peptides that derived from cytoplasmic amphipathic helical peptides of M2 proteins of different type A virus strains all bind to cultured cells. An indication that the homologous regions in all type A influenza virus M2 proteins share the similar property of inducing fusion protein internalization. To identify other amphipathic helix peptides that also share the similar property of binding to the cell and even of being internalized into the cell, the inventor searched the literature to identify amphipathic helix peptides that had been shown to interact with the membrane when fused to a GFP reporter. Through this approach, the inventor identified three amphipathic helix peptides that met our criteria, HAfp23, Grk5 and Nsp1. HAfp23 are derived from the hemagglutinin fusion domain, an amphipathic helical-hairpin that is known to induce membrane curvature. Grk5 peptides are derived from the amino acid residues 546-565 of G-protein coupled protein kinase 5 (Grk5), it has been shown to form an amphipathic helix and is sufficient for the targeting of Grk5 protein to the plasma membrane. Nsp1 is derived from the amino acid residues 245-264 of the Semliki Forest virus replicase protein nsP1, also shown to form amphipathic helix and required for the membrane targeting of nsP1. Using genetic recombination, the inventor recombined the coding sequences of these three peptides in front of the GFP coding sequence in pET28a vector. The inventor expressed and purified these three GFP fusion proteins and used them in cell binding assay to test their capacity in binding to cultured cells. The result shows among these three fusion proteins, only Grk5-GFP is able to bind to the cultured cells with about 30% of the activity of AH2-GFP. HAfp23-GFP and Nsp1-GFP failed to bind to the cultured cells in this assay (FIG. 9B).

### Example 10

Comparing the amphipathic helical peptide sequences of AH2, AH3, AH5 and Grk5, the inventor has identified a function motif with a consensus sequence of R-L/I/R-F-K. And there is another similar motif in the amino acid 2-6 of AH2, AH3 and AH5: R-L-F-S/F-K. To test if this motif is important for the binding and internalization of amphipathic helical peptide into the cell, the viral protein database is searched for the possible combinations of this motif. The identified proteins that contain this motif were further evaluated using a secondary structure prediction program. Only those proteins that contain this motif in a helical structure were chosen. Four peptide sequences containing these RLFK or RLFFK motifs are ANMV-AH, SWS-AH, TYLCV-AH and AHV-AH. Peptides with corresponding sequences were synthesized and labeled with FITC. These peptides were used for assay to test if the presence of this motif is sufficient for cell binding and internalization. In the presence of same peptide concentrations, AHV-AH, SWS-AH, ANMV-AH, TYLCV-AH peptides bind to cells with an activity of 40%, 24%, 15% and 25%, respectively, when compared to AH2 peptide (FIG 10B). This result suggests that the presence of RLFK/RLFFK motif in an amphipathic helical structure is sufficient to enable binding to the membrane and internalization of an amphipathic helical peptide as well as its fusion antigen.

### Example 11

Following internalization, the protein fused with RLFFK/RLFK motif containing peptides is recycled back to the media. The EM result indicates that the AH2 peptide induced membrane vehicle may fuse with the plasma membrane and cause the release of a lipo-protein complex. To test this possibility, GFP fused with AH2, GRK5 or SWS amphipathic peptides were incubated with cultured MDCK cells for 4 hours and then washed with PBS before being digested with thrombin to remove GFP from the extracellular fusion protein. After digestion, cells were washed and cultured in growth media for an additional 24 hours. The fluorescent level of GFP fusion proteins released from the cells was measured by a fluorometer. The result suggests there are still 40%, 45% and 30% of fusion protein remaining in the cells after 24 hours incubation (FIG. 11A). And the fusion protein released from the cells can be identified in the culturing media (FIG. 11B).

### Example 12

The polarization of AH2 peptide mediated immune stimulation toward Th2 responses. Also to evaluate whether the immune response stimulated by peptide containing RLFFK/RLFK motif is Helper T cell 1 (Th1) or Th2 biased, the titer of anti-GFP IgG or IgG subclasses after immunization were determined by ELISA. AH1 peptides that do not contain RLFFK/RLFK motif are used for comparison. His-GFP, AH1-GFP and AH2-GFP fusion proteins were purified and intramuscularly injected into BALB/c strain mice at day 0 and day 14 to induce immune responses. Sera collected at day 28 were used for ELISA analysis using either Goat anti-mouse total IgG (FIG. 12A) or anti-IgG1, anti-IgG2a, anti-IgG2b and anti-IgG3 (FIG. 12B). By comparing the IgG1 vs. IgG2a+IgG3 ratio, the result suggests that the immune responses triggered by AH2-GFP are Th2 biased, which indicates that the AH2 peptide may trigger T-cell dependent humoral immunity.

To test whether the presence of the RLFK motif in an amphipathic helical peptide is sufficient to stimulate a Th2 type T cell response when fused to an antigen, SWS-GFP was expressed and purified for mice immunization. On day 14 post immunization, the sera were collected from immunized mice and the titers of anti-GFP IgG and IgG subclasses were determined by ELISA. The result in FIG. 12C suggests that the IgG subclass induced by SWS-GFP is mainly IgG1, an indication that the Th2 T cell responses are activated by the addition of SWS-AH peptide that contains the RLFK motif. These results provide strong evidence that the RLFK/RLFFK motif in an amphipathic helical peptide is able to deliver antigen to the endosomal pathway in an antigen presenting cell and induce T cell dependent humoral immunity.

While particular embodiments of the present invention have been shown and described, it will be obvious to those skilled in the art that, based upon the teachings herein, changes and modifications may be made without departing from this invention and its broader aspects. The appended claims are intended to encompass within their scope all such changes and modifications as being within the true spirit and scope of the exemplary embodiment(s) of the present invention.

## Claims

1. A method of enhancing immune response, comprising:
combining an amphipathic peptide to an antigen to form an antigen-complex composition; and
administering the antigen-complex into a subject.

2. The method according to claim 1, wherein the amphipathic peptide ranges from 4 to 45 amino acids.

3. The method according to claim 2, wherein the amphipathic peptide comprises an amino acid sequence of SEQ ID NO: 1, an amino acid sequence of SEQ ID NO: 2, or an amino acid sequence of SEQ ID NO: 3.

4. The method according to claim 2, wherein the amphipathic peptide comprises an amino acid sequence including an A₁B₁B₂A₂ and/or A₃B₃B₄B₅A₄ motif,
wherein,
A₁, A₂, A₃ and A₄ are each individually selected from the group consisting of arginine (R) and lysine (K);
B₁ is selected from the group consisting of lysine (K), arginine (R), isoleucine (I), leucine (L), and valine (V);
B₂ is selected from the group consisting of isoleucine (I), leucine (L), valine (V), phenylalanine (F), tyrosine (Y), and tryptophan (W);
B₃ is selected from the group consisting of isoleucine (I), leucine (L), and valine (V);
B₄ is selected form the group consisting of phenylalanine (F), tryptophan (W) and tyrosine (Y); and
B₅ is selected from the group consisting of phenylalanine (F), tyrosine (Y), tryptophan (W), threonine (T), cysteine (C) and serine (S).

5. The method according to claim 4, wherein the amphipathic peptide comprises an amino acid sequence of SEQ ID NO: 4, an amino acid sequence of SEQ ID NO: 5, an amino acid sequence of SEQ ID NO: 6, an amino acid sequence of SEQ ID NO: 7, an amino acid sequence of SEQ ID NO:8, an amino acid sequence of SEQ ID NO: 9, an amino acid sequence of SEQ ID NO: 10 or an amino acid sequence of SEQ ID NO: 11.

6. The method according to claim 1, wherein the antigen is a part of a pathogenic agent selected from the group consisting of Influenza virus of Type A, Influenza virus of Type B, Influenza virus of Type C, Dengue virus, Human papillomavirus, Hepatitis A virus, Hepatitis B virus, Hepatitis C virus, Varicella Zoster virus, Cytomegalovirus, Ebola virus, *Haemophilus* influenza type b, *Helicobacter pylori,* Herpes simplex virus 1 and 2, *Plasmodium* species, Measles virus, Middle East respiratory syndrome coronavirus, Mumps virus, *Bordetella pertussis,* Poliovirus, Rabies virus, Respiratory syncytial virus, Rotavirus, Rubella virus, SARS coronavirus, *Clostridium tetani, Mycobacterium tuberculosis,* West Nile virus, *Streptococcus pneumoniae, Staphylococcus aureus, Clostridium difficile, Neisseria meningitidis* and *Salmonella enterica* subsp. *enterica.*

7. The method according to the claim 1, wherein the antigen is selected from the group consisting of an oligosaccharide, polypeptide, protein, polysaccharide, peptide and a combination thereof.

8. The method according to the claim 1, wherein the subject is warm-blooded animals.

9. An antigen-complex composition, comprising:
an A₁B₁B₂A₂ and/or A₃B₃B₄B₅A₄ motif;
a first peptide, linking to the N-terminal of the A₁B₁B₂A₂ and/or A₃B₃B₄B₅A₄ motif;
a second peptide, linking to the C-terminal of the A₁B₁B₂A₂ and/or A₃B₃B₄B₅A₄ motif; and
an antigen, covalent linked to the first peptide or to the second peptide,
wherein,
A₁, A₂, A₃ and A₄ are each individually selected from the group consisting of arginine (R) and lysine (K);
B₁ is selected from the group consisting of lysine (K), arginine (R), isoleucine (I), leucine (L), and valine (V);
B₂ is selected from the group consisting of isoleucine (I), leucine (L), valine (V), phenylalanine (F), tyrosine (Y), and tryptophan (W);
B₃ is selected from the group consisting of, isoleucine (I), leucine (L), and valine (V);
B₄ is selected form the group consisting of phenylalanine (F), tryptophan (W) and tyrosine (Y); and
B₅ is selected from the group consisting of phenylalanine (F), tyrosine (Y), tryptophan (W), threonine (T), cysteine (C) and serine (S); and
the A₁B₁B₂A₂ and/or A₃B₃B₄B₅A₄ motif, the first peptide and the second peptide form an amphipathic helix secondary structure.

10. The antigen-complex composition according to claim 9, wherein the first peptide ranges from 0 to 20 amino acids and the second peptide ranges from 0 to 20 amino acids.

11. The antigen-complex composition according to claim 9, wherein the antigen is selected from the group consisting of an oligosaccharide, polypeptide, protein, polysaccharide, peptide and a combination thereof.

12. A method of improving antigen delivery, comprising:
administering an antigen-complex composition to a subject,
wherein the antigen-complex composition comprises:
an A₁B₁B₂A₂ and/or A₃B₃B₄B₅A₄ motif;
a first peptide, linking to the N-terminal of the A₁B₁B₂A₂ and/or A₃B₃B₄B₅A₄ motif;
a second peptide, linking to the C-terminal of the A₁B₁B₂A₂ and/or A₃B₃B₄B₅A₄ motif; and
an antigen, covalently linked to the first peptide or to the second peptide,
wherein:
A₁, A₂, A₃ and A₄ are each individually selected from the group consisting of arginine (R) and lysine (K);
B₁ is selected from the group consisting of lysine (K), arginine (R), isoleucine (I), leucine (L), and valine (V);
B₂ is selected from the group consisting of isoleucine (I), leucine (L), valine (V), phenylalanine (F), tyrosine (Y), and tryptophan (W);
B₃ is selected from the group consisting of, isoleucine (I), leucine (L), and valine (V);
B₄ is selected form the group consisting of phenylalanine (F), tryptophan (W) and tyrosine (Y); and
B₅ is selected from the group consisting of phenylalanine (F), tyrosine (Y), tryptophan (W), threonine (T), cysteine (C) and serine (S); and
the A₁B₁B₂A₂ and/or A₃B₃B₄B₅A₄ motif, the first peptide and the second peptide form an amphipathic helix secondary structure.

13. The method according to claim 12, wherein the first peptide ranges from 0 to 20 amino acids and the second peptide ranges from 0 to 20 amino acids.

14. The method according to claim 12, wherein the antigen is selected from the group consisting of an oligosaccharide, polypeptide, protein, polysaccharide, peptide and a combination thereof.

15. An amphipathic peptide comprises an amino acid sequence of SEQ ID NO: 1, an amino acid sequence of SEQ ID NO: 2, an amino acid sequence of SEQ ID NO: 3, an amino acid sequence of SEQ ID NO: 4, an amino acid sequence of SEQ ID NO: 5, an amino acid sequence of SEQ ID NO: 6, an amino acid sequence of SEQ ID NO: 7, an amino acid sequence of SEQ ID NO: 8, an amino acid sequence of SEQ ID NO: 9, an amino acid sequence of SEQ ID NO: 10, or an amino acid sequence of SEQ ID NO: 11.


SEQUENCE LISTING

<110>  Vaxsia Biomedical Inc.

<120>  NOVEL PROTEIN STRUCTURE USED FOR EFFICIENT ANTIBODY PRODUCTION IN
IMMUNIZATION

<150>  62/090,180
<151>  2014-12-10

<160>  11

<170>  PatentIn version 3.5

<210>  1
<211>  15
<212>  PRT
<213>  Hepatitis C virus

<400>  1

Thr Arg Gly Leu Leu Gly Cys Ile Ile Thr Ser Leu Thr Gly Arg
1               5                   10                  15

<210>  2
<211>  20
<212>  PRT
<213>  Semliki Forest virus

<400>  2

Gly Ser Thr Leu Tyr Thr Glu Ser Arg Lys Leu Leu Arg Ser Trp His
1               5                   10                  15

Leu Pro Ser Val
            20

<210>  3
<211>  23
<212>  PRT
<213>  Artificial sequence

<220>
<223>  amphipathic helix

<400>  3

Gly Leu Phe Gly Ala Ile Ala Gly Phe Ile Glu Gly Gly Trp Thr Gly
1               5                   10                  15

Met Ile Asp Gly Trp Tyr Gly
            20

<210>  4
<211>  19

<212>  PRT
<213>  Influenza A virus, H1N1

<400>  4

Asp Arg Leu Phe Ser Lys Ser Ile Tyr Arg Ile Phe Lys His Gly Leu
1               5                   10                  15

Lys Arg Gly

<210>  5
<211>  19
<212>  PRT
<213>  Influenza A virus, H5N1

<400>  5

Asp Arg Leu Phe Phe Lys Cys Ile Tyr Arg Arg Leu Lys Tyr Gly Leu
1               5                   10                  15

Lys Arg Gly

<210>  6
<211>  19
<212>  PRT
<213>  Influenza A virus, H7N9

<400>  6

Asp Arg Leu Phe Phe Lys Cys Ile Tyr Arg Arg Phe Lys Tyr Gly Leu
1               5                   10                  15

Lys Arg Gly

<210>  7
<211>  20
<212>  PRT
<213>  Artificial sequence

<220>
<223>  amphipathic helix

<400>  7

Pro Lys Lys Gly Leu Leu Gln Arg Leu Phe Lys Arg Gln His Gln Asn
1               5                   10                  15

Asn Ser Lys Ser
            20

<210>  8
<211>  18
<212>  PRT
<213>  Shrimp white spot syndrome virus

<400>  8

Lys Arg Leu Phe Lys Leu Ser Tyr Ile Glu Thr Arg Tyr Met Ala Ser
1               5                   10                  15

Asp Lys

<210>  9
<211>  18
<212>  PRT
<213>  Tomato yellow leaf curl virus

<400>  9

Met Pro Arg Leu Phe Lys Ile Tyr Ala Lys Asn Tyr Phe Leu Thr Tyr
1               5                   10                  15

Pro Asn

<210>  10
<211>  18
<212>  PRT
<213>  Ateline herpesvirus 3

<400>  10

Arg Thr Leu Ser Leu Leu Arg Arg Leu Phe Phe Lys Ser Asp Leu Gly
1               5                   10                  15

Phe Leu

<210>  11
<211>  18
<212>  PRT
<213>  ANMV-1 virus

<400>  11

Met His Ser Ala Thr Ala Ala Gln Leu Glu Thr Leu Arg Leu Phe Lys
1               5                   10                  15

Phe Pro